# EUROPEAN PATENT APPLICATION

(11) **EP 2 239 265 A1**
(43) Date of publication of application: **13.10.2010**
(21) Application number: 09706908.2
(22) Date of filing: 26.01.2009
(51) Int. Cl.: C07F 9/6558, C07C 57/145, C07C 57/15, A61K 31/675, A61P 25/28, A61P 43/00

(54) **CRYSTALLINE CINNAMIDE COMPOUNDS OR SALTS THEREOF**

(30) Priority: 28.01.2008 JP 2008016692
(71) Applicant: Eisai R&D Management Co., Ltd., Tokyo 112-8088 (JP)
(72) Inventor: KUSHIDA, Ikuo, Tsukuba-shi Ibaraki 300-2635 (JP); SATO, Nobuaki, Tsukuba-shi Ibaraki 300-2635 (JP); SATO, Yoshiaki, Tsukuba-shi Ibaraki 300-2635 (JP)
(74) Representative: HOFFMANN EITLE
(86) International application number: PCT/JP2009/051162
(87) International publication number: WO 2009/096349

(57) **Abstract**

Disclosed are crystals of the compound represented by Formula (1), which has Aβ-production-reducing effects and is a prodrug of compounds effective in treating neural degenerative diseases of which Aβ is the cause, for example, diseases such as Alzheimer's disease, Down syndrome, etc., and salts thereof or crystals of salts thereof. These are particularly useful as stock for pharmaceutical products.

## Description

### TECHNICAL FIELD

The present invention relates to crystalline cinnamide compounds having an amyloidβ production reducing effect or salts thereof

### BACKGROUND ART

Alzheimer's disease is a disease characterized by the formation of senile plaques and neurofibrillary tangle along with the degeneration and drop-out of the neuron. The current treatment for Alzheimer's disease is limited to a symptomatic treatment using a symptom alleviating drug represented by an acetylcholinesterase inhibitor, and a medication for definitive care to delay the progression of the disease has yet been developed. To create a medication for the definitive care of Alzheimer's disease, it is essential to develop a method for controlling the cause of the onset of the disease.
The Aβ protein, a metabolite of amyloid precursor protein (hereinafter referred to as APP), is considered to have been greatly involved in the degeneration and drop-out of the neuron and further in the onset of dementia symptoms (see e.g., Non-patent Documents 1 and 2). The main components of the Aβ protein are Aβ40 consisting of 40 amino acids and Aβ42 having 2 more amino acids at the C-terminal. These Aβ 40 and Aβ 42 are highly aggregative (see e.g., Non-patent Document 3) and are the main components of senile plaques (see e.g., Non-patent Documents 4 and 5), and it is further known that the mutations of APP and presenilin genes observed in familial Alzheimer's disease increase these Aβ 40 and Aβ 42 (see e.g., Non-patent Documents 6, 7 and 8). Thus, a compound capable of reducing the production of Aβ 40 and Aβ 42 is expected to be an agent to suppress progress or prevent Alzheimer's disease.
Non-patent Document 1: Klein WL and 7 others, Alzheimer's disease-affected brain: Presence of oligomeric Aβ ligands (ADDLs) suggests a molecular basis for reversible memory loss, Proceding National Academy of Science USA 2003, Sep 2; 100(18), p. 10417-10422.
Non-patent Document 2: Nitsch RM and 16 others, Antibodies against β-amyloid slow cognitive decline in Alzheimer's disease, Neuron, 2003, May 22; 38, p.547-554.
Non-patent Document 3: Jarrett JT and 2 others, the carboxy terminus of the β amyloid protein is critical for the seeding of amyloid formation: Implications for the pathogenesis of Alzheimers' disease, Biochemistry, 1993, 32(18), p.4693-4697.
Non-patent Document 4: Glenner GG and 1 other, Alzheimer's disease: initial report of the purification and characterization of a novel cerebrovascular amyloid protein, Biochemical and biophysical research communications, 1984, May 16, 120(3), p.885-890.
Non-patent Document 5: Masters CL and 5 others, Amyloid plaque core protein in Alzheimer disease and Down syndrome, Proceding National Academy of Science USA, 1985, Jun, 82(12), p.4245-4249.
Non-patent Document 6: Gouras GK and 11 others, Intraneuronal Aβ 42 accumulation in human brain, American Journal of Pathology, 2000, Jan, 156(1), p.15-20.
Non-patent Document 7: Scheuner D and 20 others, Secreted amyloid β-protein similar to that in the senile plaques of Alzheimer's disease is increased in vivo by the presenilin 1 and 2 and APP mutations linked to familial Alzheimer's disease, Nature Medicine, 1996, Aug, 2(8), p.864-870. Non-patent Document 8: Forman MS and 4 others, Differential effects of the swedish mutant amyloid precursor protein on β-amyloid accumulation and secretion in neurons and nonneuronal cells, The Journal of Biological Chemistry, 1997, Dec 19, 272(51), p.32247-32253.

### DISCLOSURE OF THE INVENTION

### PROBLEMS TO BE SOLVED BY THE INVENTION

The properties of compounds or salts thereof, or crystals thereof useful as pharmaceutical products greatly affect the bioavailability of a drug, the purity of a bulk drug substance, the formulation of a preparation, etc. For this reason, it is required to study which salts and crystal forms of such a compound are best suited as a pharmaceutical product in the development of pharmaceutical products. More specifically, since these properties depend on the attribution of individual compound, it is generally difficult to predict a salt and crystal form having suitable properties for a bulk drug substance and hence each compound practically needs to be examined in a various way.

### MEANS FOR SOLVING THE PROBLEMS

The present inventors have found that amorphous 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate (hereinafter referred to as compound (1)) represented by the following formula 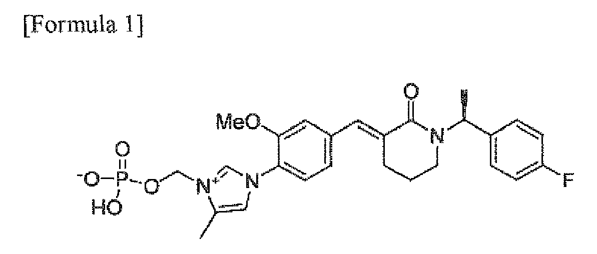 has an Aβ production reducing effect and is hence useful as a prodrug of (E)-1-[(1S)-1-(4-fluorophenyl)ethyl]-3-[3-methoxy-4-(4-methyl-1H-imidazol-1-yl)benzylidene]piperidin-2-one, a compound effective to treat neurodegenerative diseases caused by Aβ such as Alzheimer's disease, or Down's syndrome (PCT/JP 07/064637). The inventors isolated various salts and crystals of the compound (1) to understand the properties and forms thereof and conducted various studies, and identified the crystals, salts and crystalline salts thereof having good physical properties for a bulk drug substance, whereby the present invention was accomplished.

More specifically, the present invention relates to
(1) a salt of 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate,
(2) the salt according to the above (1) which is an organic acid salt,
(3) the salt according to the above (1) or (2) which is fumarate,
(4) the salt according to the above (1) or (2) which is maleate,
(5) the salt according to the above (1) which is an inorganic acid salt,
(6) the salt according to the above (1) or (5) which is hydrobromate,
(7) a crystal of fumarate according to the above (3) having a peak at a diffraction angle (2θ±0.2°) of 11.3°, 19.0° and/or 23.2° in the X-ray powder diffraction peak,
(8) a crystal of maleate according to the above (4) having a peak at a diffraction angle (2θ±0.2°) of 5.9°, 14.7° and/or 19.4° in the X-ray powder diffraction peak,
(9) a crystal of hydrobromate according to the above (6) having a peak at a diffraction angle (2θ±0.2°) of 9.3°, 19.9° and/or 21.8° in the X-ray powder diffraction peak,
(10) a crystal (I) of 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate having a peak at a diffraction angle (2θ±0.2°) of 11.1°, 15.5° and/or 19.5° in the X-ray powder diffraction peak,
(11) a crystal (II) of 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate having a peak at a diffraction angle (2θ±0.2°) of 6.0°, 15.3° and/or 24.1° in the X-ray powder diffraction peak,
(12) a crystal (III) of 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate having a peak at a diffraction angle (2θ±0.2°) of 6.7°, 13.5° and/or 16.1° in the X-ray powder diffraction peak,
(13) a crystal (IV) of 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate having a peak at a diffraction angle (2θ±0.2°) of 7.1°, 11.7° and/or 14.2° in the X-ray powder diffraction peak, and
(14) a crystal (V) of 1-{4-[(E)-{1-[(1S)-1-(4-fluorophenyl)ethyl]-2-oxopiperidin-3-y!idene}methyl]-2-methoxyphenyl}-4-methyl-1H-imidazol-3-iomethyl monohydrogen phosphate having a peak at a diffraction angle (2θ±0.2°) of 6.3°, 6.7° and/or 14.2° in the X-ray powder diffraction peak, and is a novel invention undisclosed in any other documents.

Hereinafter, the content of the present invention is described in detail.

The compound (1) of the present invention can be produced by the synthesis procedure described in Production Examples to be described later.

The salt of the compound (1) of the present invention is not limited insofar as it is a salt formed with the compound (1) and is a pharmacologically acceptable. Examples include salts with inorganic acids, organic acids, inorganic bases, organic bases, acidic or basic amino acids, salts with inorganic acids and organic acids being preferable. A hydrate of a salt is also encompassed in the scope of the present invention.
Preferable examples of the inorganic acid salt include hydrochloride, hydrobromate, sulphate, nitrate, and phosphate, hydrochloride, hydrobromate, sulphate and phosphate being more preferable, and hydrobromate being the most preferable.
Preferable examples ofthe organic acid salt include acetate, succinate, fumarate, maleate, tartrate, citrate, lactate, stearate, benzoate, methanesulfonate, ethanesulfonate, p-toluenesulfonate, and benzenesulfate, fumarate and maleate being particularly preferable.
Preferable examples of the inorganic base salt include alkali metal salts such as sodium salt and potassium salt, alkaline earth metal salts such as calcium salt and magnesium salt, aluminium salt, and ammonium salt.
Preferable examples of the organic base salt include diethylamine salt, diethanolamine salt, meglumine salt, and N,N'-dibenzylethylenediamine salt.
Preferable examples of the acidic amino acid salt include aspartate and glutamate, and preferable examples of the basic amino acid salt include arginine salt, lysine salt, and ornithine salt.

A crystal of the salt of the compound (1) can be produced by dissolving the compound (1) and a predetermined acid or base in a solvent and precipitating the salt from the solution. More specifically, the compound (1) and a solvent are mixed at room temperature or under heating, and a predetermined acid or base is further added thereto and dissolved. The solution is gradually cooled to 4°C to room temperature to precipitate the salt.

A crystal of the compound (1) can be produced by dissolving the compound (1) in a solvent and precipitating the crystal. More specifically, the compound (1) is added to a solvent at room temperature or under heating, stirred and dissolved. The solution is gradually cooled to 0 to 60°C, preferably 4°C to room temperature, to precipitate the crystal.

The solvent used for producing the salt crystal of the compound (1) is not limited insofar as it dissolves the compound (1) and a predetermined acid or base. For producing the crystal of the compound (1), the solvent is neither limited insofar as it dissolves the compound (1). Either for producing the salt crystal of the compound (1) or the crystal ofthe compound (1), preferable solvents to be used include methanol, 1-propanol, 2-butanone, acetone, toluene, acetonitrile and mixed solvents thereof. For producing the crystal of the compound (1), the solvents also usable include water and mixtures of water and these solvents. Either for producing the salt crystal of the compound (1) or the crystal of the compound (1), the amount of the solvent is not limited and can be suitably selected from an amount in which the compound (1) is dissolved by heating as being the lower limit to an amount in which the yield of the crystal is not significantly decreased as being the upper limit.
Either for producing the salt crystal of the compound (1) or the crystal of the compound (1), the heating temperature may be suitably selected from the temperatures at which the compound (1) is dissolved, but preferably from 10°C to the reflux temperature of the solvent, more preferably from room temperature to 60°C. The slow cooling can be carried out at a rate of 30 to 5°C/hr, but natural cooling is preferable.
The acid or base for producing a crystal of the salt of the compound (1) can be used in an equivalent amount of 0.1 to 10 to the compound (1). More specifically, the salt crystal of the compound (1) can be produced in accordance with the procedures shown in Examples below.

The compound (1) used in the above production process may be an anhydrous form or a hydrate, any crystal or amorphous solid, or a mixture thereof.

The crystallized crystal is separated by a common filtering operation, washed using a solvent as necessary, and further dried to obtain the intended crystal. The solvent used for washing the crystal may be the same as a crystallization solvent but may be a different solvent. The crystal can be dried by being left under the air or also be dried by heating. Alternatively, the crystal can be dried under ventilation or under a reduced pressure.

The diffraction angle (2θ) in the X-ray powder diffraction of the crystal of the present invention may contain errors within the range of the diffraction angle ±0.2°. For this reason, the values of the diffraction angle shown in the present specification should be understood as those containing numerical values within a range of ±0.2°. Thus, the present invention encompasses not only the crystals having peak diffraction angles that completely match in the X-ray powder diffraction but also the crystals having peak diffraction angles that match within an error of ±0.2°.

Taking the crystal of fumarate as an example for more specific description, the "having a diffraction peak at a diffraction angle (2θ±0.2°) of 11.3°" in the present specification means "having a diffraction peak at diffraction angles (2θ) within a range from 11.1° ° to 11.5°." Further, the "having a diffraction peak at a diffraction angle (2θ±0.2°) of α°, β° and/or γ°" means to have at least one diffraction peak among the above diffraction peaks.

In the present invention, preferable crystals of salts of the compound (1) are particularly a crystal of fumarate, a crystal of maleate and a crystal of hydrobromate. Particularly preferable among those are the crystal of fumarate having a peak at a diffraction angle (2θ±0.2°) of 11.3°, 19.0° and/or 23.2° in the X-ray powder diffraction peak; the crystal of maleate having a peak at a diffraction angle (2θ±0.2°) of 5.9°, 14.7° and/or 19.4°; and the crystal of hydrobromate having a peak at a diffraction angle (2θ±0.2°) of 9.3°, 19.9° and/or 21.8° in the X-ray powder diffraction peak.

Preferable crystals ofthe compound (1) are particularly the crystal (I) having a peak at a diffraction angle (2θ±0.2°) of 11.1°, 15.5° and/or 19.5° in the X-ray powder diffraction peak; the crystal (II) having a peak at a diffraction angle (2θ±0.2°) of 6.0°, 15.3° and/or 24.1° in the X-ray powder diffraction peak; the crystal (III) having a peak at a diffraction angle (2θ±0.2°) of 6.7°, 13.5° and/or 16.1° in the X-ray powder diffraction peak; the crystal (IV) having a peak at a diffraction angle (2θ±0.2°) of 7.1°, 11.7° and/or 14.2° in the X-ray powder diffraction peak; and the crystal (V) having a peak at a diffraction angle (2θ±0.2°) of 6.3°, 6.7° and/or 14.2° in the X-ray powder diffraction peak.

When the crystal of the compound (1) of the present invention, the salt thereof or the salt crystal thereof is used as a pharmaceutical product, the crystal of the compound (1) of the present invention, the salt thereof or the crystal of the salt thereof is typically mixed with suitable additives and formulated for use. However, this description does not mean to exclude the use of the crystal of the compound (1) or the crystal of the salt of the compound (1) of the present invention as a bulk drug substance for pharmaceuticals.
The above additives, which are commonly used for pharmaceuticals, include excipients, binders, lubricants, disintegrators, colorants, flavoring agents, emulsifiers, surfactants, solubilizing agents, suspending agents, tonicity adjusting agents, buffers, antiseptics and antioxidants, and these additives can be used in suitable combination as desired.

Examples of the above excipient include lactose, saccharose, glucose, cornstarch, mannitol, sorbitol, starch, pregelatinized starch, dextrin, crystalline cellulose, light anhydrous silicic acid, aluminium silicate, calcium silicate, magnesium aluminometasilicate and dibasic calcium phosphate.
Examples of the above binder include polyvinyl alcohol, methylcellulose, ethylcellulose, gum arabic, tragacanth, gelatin, shellac, hydroxypropylmethyl cellulose, hydroxypropylcellulose, sodium carboxymethyl cellulose, polyvinylpyrrolidone and macrogoal.
Examples of the above lubricant include magnesium stearate, calcium stearate, sodium stearyl fumarate, talc, polyethylene glycol and colloidal silica.
Examples of the above disintegrator include crystalline cellulose, agar agar, gelatin, calcium carbonate, sodium bicarbonate, calcium citrate, dextrin, pectin, low-substituted hydroxypropyl cellulose, carboxymethyl cellulose, calcium carboxymethyl cellulose, croscarmellose sodium and carboxymethyl starch and sodium carboxymethyl starch.
Examples of the above colorant include colorants accepted to be added to pharmaceutical products such as iron sesquioxide, yellow iron sesquioxide, carmine, caramel, β-carotene, titanium oxide, talc, riboflavin sodium phosphate and yellow aluminium lake.
Examples of the above flavoring agent include cocoa powder, menthol, aromatic powder, peppermint oil, borneo camphor and cinnamomi cortex pulveratus.
Examples of the above emulsifier or surfactant include stearyl triethanolamine, sodium lauryl sulfate, lauryl aminopropionate, lecithin, glyceryl monostearate, sucrose fatty acid ester and glycerine fatty acid ester,
Examples of the above solubilizing agent include polyethylene glycol, propylene glycol, benzyl benzoate, ethanol, cholesterol, triethanolamine, sodium carbonate, sodium citrate, polysorbate 80 and nicotinamide.
Examples of the above suspending include, in addition to the above surfactants, hydrophilic polymers such as polyvinyl alcohol, polyvinylpyrrolidone, methyl cellulose, hydroxymethylcellulose, hydroxyethylcellulose and hydroxypropylcellulose.
Examples of the above tonicity adjusting agent include glucose, sodium chloride, mannitol and sorbitol.
Examples of the above buffer include buffer solutions containing phosphate, acetate, carbonate, citrate, and like.
Examples of the above antiseptic include methylparaben, propylparaben, chlorobutanol, benzyl alcohol, phenethyl alcohol, dehydroacetic acid and sorbic acid.
Examples of the above antioxidant include sulfite, ascorbic acid and α-tocopherol.

Further, examples ofthe formulations mentioned earlier include oral preparations such as tablets, powders, granules, capsules, syrups, troches and inhalants; external preparations such as suppositories, ointments, ophthalmic ointments, plasters, ophthalmic solutions, nasal drops, ear drops, cataplasms and lotions; and injections.
The above oral preparations are formulated in combination with the above additives as necessary. The oral preparations may further be surface-coated as necessary.
The above external preparations are formulated, as necessary, in combination with, among the above additives, particularly, an excipient, a binder, a flavoring agent, an emulsifier, a surfactant, a solubilizing agent, a suspending, a tonicity adjusting agent, an antiseptic, antioxidant, a stabilizer or an absorption promoting agent.
The above injections are formulated, as necessary, in combination with, among the above additives, particularly, an emulsifier, a surfactant, a solubilizing agent, a suspending, a tonicity adjusting agent, a buffer, an antiseptic, an antioxidant, a stabilizer or an absorption promoting agent.

The dosage of the pharmaceutical of the present invention varies depending on severity of conditions, age, sex, body weight, administration form, type of salt, sensibility difference to a drug, specific type of disease, and the like, but is typically about 30 µg to 10 g (preferably 1 mg to 1 g) of the crystal of the compound (1), the salt thereof or the crystal of the salt thereof a day per adult in an oral administration; 30 µg to 20 g (preferably 100 µg to 10 g) of the crystal of the compound (1), the salt thereof or the salt crystal thereof in the form of external preparation; and 30 µg to 1 g (preferably 100 µg to 500 mg) of the crystal of the compound (1) or the crystal of the salt thereof a day in a single administration or two to six divided administrations in the form of injection preparation.

### ADVANTAGES OF THE INVENTION

The crystal of the compound (1) of the present invention, the salt thereof or the crystal of the salt thereof have good physical properties and an Aβ production reducing effect and are suitably used as active substance of a therapeutic agent or a preventive agent effective for treating neurodegenerative diseases caused by Aβ such as Alzheimer's disease, or Down's syndrome, and are also suitably used as bulk drug substances for these pharmaceutical products.

### BEST MODE FOR CARRYING OUT THE INVENTION

The compound (1) of the present invention, the crystal of the compound (1) and the crystal of the salt of the compound (1) can be produced by, for example, the processes described in the following Production Examples and Examples. These are only examples, however and the compound of the present invention is not limited to the following specific examples in any case whatsoever.

### Production Example 1

### 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate trifluoroacetate

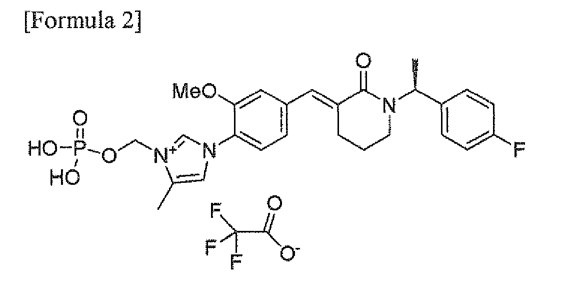

In a nitrogen atmosphere, (E)-1-[(1S)-1-(4-fluorophenyl)ethyl]-3-[3-methoxy-4-(4-methyl-1H-imidazol-1-yl)benzylidene]piperidin-2-one (CAS #870843-42-8, 200 mg) was added to an acetone solution (4 mL) of chloromethyl di-tert-butylphosphate (CAS No. 229625-50-7, 185 mg), sodium iodide (214 mg) and diisopropylethylamine (21 µL). The reaction solution was stirred at 60°C for 1 hour. The reaction solution was concentrated under reduced pressure, and methylene chloride (0.2 mL) and trifluoroacetic acid (0.3 mL) were added to the obtained residue. The resulting solution was stirred at room temperature for 2.5 hours. The reaction solution was concentrated. A 25% acetonitrile aqueous solution of the obtained residue was subjected to reversed phase C18 silica gel column chromatography (developing solvent: 30% acetonitrile aqueous solution containing 0.1% trifluoroacetic acid). The objective fraction was concentrated and lyophilized to give 247 mg of the title compound.
¹H-NMR (400 MHz, CD₃OD) δ (ppm): 1.58 (d, J=7.2 Hz, 3H), 1.63-1.90 (m, 2H), 2.53 (s, 3H), 2.80-2.90 (m, 2H), 2.95-3.05 (m, 1H), 3.35-3.42 (m, 1H), 3.92 (s, 3H), 5.94 (d, J=12.8 Hz, 2H), 6.08 (q, J=7.2 Hz, 1H), 7.10 (t, J=8.8 Hz, 2H), 7.22 (d, J=8.0, 1H), 7.32 (s, 1H), 7.38 (dd, J=8.8, 5.2 Hz, 2H), 7.58 (d, J=8.0, 1H), 7.68 (d, J=1.6 Hz, 1H), 7.80 (s, 1H), 9.42 (d, J=1.6 Hz, 1H).

### Production Example 2

### 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate

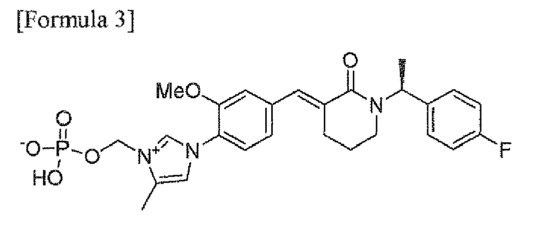

An aqueous solution (4 mL) of the 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate trifluoroacetate (150 mg) obtained in Production Example 1 was subjected to reversed phase C18 silica gel column chromatography (developing solvent: water → 35% acetonitrile aqueous solution). The objective fraction was concentrated and lyophilized to give 112 mg of the title compound.
¹H-NMR (400 MHz, CD₃OD) δ (ppm): 1.59 (d, J=7.2 Hz, 3H), 1.63-1.90 (m, 2H), 2.53 (s, 3H), 2.78-2.85 (m, 2H), 2.95-3.04 (m, 1H), 3.35-3.42 (m, 1H), 3.96 (s, 3H), 5.88 (d, J=12.8 Hz, 2H), 6.09 (q, J=7.2 Hz, 1H), 7.10 (t, J=8.8 Hz, 2H), 7.21 (dd, J=8.0, 1.2 Hz, 1H), 7.31 (d, J=1.2 Hz, 1H), 7.38 (dd, J=8.8, 5.2 Hz, 2H), 7.59 (d, J=8.0 Hz, 1H), 7.65 (d, J=1.6 Hz, 1H), 7.80 (s, 1H), 9.38 (d, J=1.6 Hz, 1H).

### Production Example 3

### 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate (alternative process)

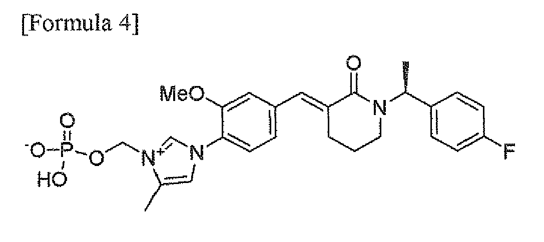

In a nitrogen atmosphere, sodium iodide (3.22 g), (E)-1-[(1S)-1-(4-fluorophenyl)ethyl]-3-[3-methoxy-4-(4-methyl-1H-imidazol-1-yl)benzylidene]piperidin-2-one (CAS #870843-42-8, 3.0 g) and diisopropylethylamine (0.31 mL) were added in the order mentioned to an acetone solution (36 mL) of chloromethyl di-tert-butylphosphate (CAS No. 229625-50-7, 2.77 g). The reaction solution was refluxed under heating for 1 hour and 50 minutes. The reaction solution was water-cooled and concentrated under reduced pressure. Ethyl acetate (40 mL), water (40 mL) and a 1 N sodium hydroxide aqueous solution (0.72 mL) were added to the obtained residue and stirred, and the water layer was partitioned. The obtained water layer was subjected to reversed phase C18 silica gel column chromatography (developing solvent: 5% acetonitrile aqueous solution → 35% acetonitrile aqueous solution). The objective fraction was concentrated and the obtained residue was crystallized using water/acetone. The crystals were obtained by filtration and dried under reduced pressure to give 3.36 g of the title compound. ¹H-NMR of the obtained product corresponded with that of the product obtained in Production Example 2.

### Example 1

### Crystal of 1-{4-[(E)-{1-[(1S)-1-(4-fluorophenyl)ethyl]-2-oxopiperidin-3-ylidene}methyl]-2-methoxyphonyl}-4-methyl-1H-imidazol-3-iomethyl monohydrogen phosphate fumarate

1 mL of a methanol solution of 0.1 N fumaric acid was added to a methanol (1.0 mL) solution of 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate (53 mg, 0.10 mmol), the solvent was then evaporated under a stream of nitrogen, toluene (2 mL) was added to the residue and the mixture was heated to 60°C. The solution was naturally cooled to room temperature and stirred, and the precipitated crystals were collected by filtration. The crystals were washed with heptane and dried under reduced pressure to give the title compound in the form of white crystal.
¹H-NMR (600 MHz, DMSO-d6) δ (ppm): 1.50 (d, J=7.0 Hz, 3H), 1.58-1.62 (m, 1H), 1.74-1.78 (m, 1H), 2.42 (s, 3H), 2.75-2.79 (m, 2H), 2.86-2.90 (m, 1H), 3.27-3.31 (m, 1H), 3.89 (s, 3H), 5.75 (d, J=12.0 Hz, 2H), 5.97 (q, J=7.0 Hz, 1H), 6.60 (s, 2H), 7.17 (dd, J=9.0, 9.0 Hz, 2H), 7.20 (dd, J=1.0, 9.0 Hz, 1H), 7.35 (dd, J=6.0, 9.0 Hz, 2H), 7.37 (d, J=1.0 Hz, 1H), 7.59 (d, J=8.0 Hz, 1H), 7.73 (s, 1H), 7.79 (s, 1H), 9.62 (d, J=2.0 Hz, 1H)

### Example 2

### Crystal of 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate maleate

1 mL of a methanol solution of 0.1 N maleic acid was added to a methanol (1.0 mL) solution of 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate (53 mg, 0.10 mmol), the solvent was then evaporated under a stream of nitrogen, toluene (2 mL) was added to the residue and the mixture was heated to 60°C. The solution was naturally cooled to room temperature and stirred, and the precipitated crystals were collected by filtration. The crystals were washed with heptane and dried under reduced pressure to give the title compound in the form of white crystal.
¹H-NMR (600 MHz, DMSO-d₆) δ (ppm): 1.50 (d, J=7.0 Hz, 3H), 1.58-1.62 (m, 1H), 1.74-1.78 (m, 1H), 2.42 (s, 3H), 2.75-2.79 (m, 2H), 2.86-2.90 (m, 1H), 3.27-3.31 (m, 1H), 3.89 (s, 3H), 5.75 (d, J=12.0 Hz, 2H), 5.97 (q, J=7.0 Hz, 1H), 6.34 (d, J=8.0 Hz, 1H), 6.37 (d, J=8.0 Hz, 1H), 7.17 (dd, J=9.0, 9.0 Hz, 2H), 7.20 (dd, J=1.0, 9.0 Hz, 1H), 7.35 (dd, J=6,0, 9.0 Hz, 2H), 7.37 (d, J=1.0Hz, 1H), 7.59 (d, J=8.0 Hz, 1H), 7.73 (s, 1H), 7.79 (s, 1H), 9.62 (d, J=2.0 Hz, 1H)

### Example 3

### Crystal of 1-{4-[(E)-{1-[(1S)-1-(4-fluorop-henyl)ethyl]-2-oxopiperidin-3-ylidene}methyl]-2-methoxyphenyl}-4-methyl-1H-imidazol-3-iomethyl monohydrogen phosphate hydrobromate

12.1 µl of a hydrobromic acid solution and methanol (1.0 mL) were added to 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate (55.5 mg, 0.10 mmol). After evaporating the solvent off under a stream of nitrogen, 2-butanone (3 mL) was added to the residue. The solution was stirred at room temperature and the precipitated crystals were collected by filtration. The crystals were washed with 2-butanone and dried under reduced pressure to give the title compound in the form of white crystal.
¹H-NMR (600 MHz, DMSO-d₆) δ (ppm): 1.50 (d, J=7.0 Hz, 3H), 1.58-1.62 (m, 1H), 1.75-1.79 (m, 1H), 2.44 (s, 3H), 2.76-2.80 (m, 2H), 2.87-2.91 (m, 1H), 3.28-3.32 (m, 1H), 3.90 (s, 3H), 5.96 (d, J=12.0 Hz, 2H), 5.98 (q, J=7.0 Hz, 1H), 7.18 (dd, J=9.0, 9.0 Hz, 2H), 7.23 (dd, J=1.0, 9.0 Hz, 1H), 7.35 (dd, J=6.0, 9.0 Hz, 2H), 7.40 (d, J=1.0 Hz, 1H), 7.60 (d, J=8.0 Hz, 1H), 7.74 (s, 1H), 7.90 (s, 1H), 9.60 (d, J=2.0 Hz, 1H)

### Example 4

### Crystal (I) of 1-{4-[(E)-{1-[(1S)-1-(4-flurorohenyl]-2-oxopiperidin-3-ylidene}methyl]-2-methoxyphenyl}-4-methyl-1H-imidazol-3-iomethyl monohydrogen phosphate

After evaporating the solvent of a methanol (1.0 mL) solution of 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate (53 mg, 0.10 mmol) under a stream of nitrogen, 2-butanone (2 mL) was added to the residue and the mixture was heated to 60°C. The solution was naturally cooled to room temperature and stirred, and the precipitated crystals were collected by filtration. The crystals were washed with heptane and dried under reduced pressure to give the title compound in the form of white crystal.
¹H-NMR (600 MHz, DMSO-d₆) δ (ppm): 1.50 (d, J=7.0 Hz, 3H), 1.58-1.62 (m, 1H), 1.74-1.78 (m, 1H), 2.41 (s, 3H), 2.75-2.79 (m, 2H), 2.86-2.90 (m, 1H), 3.27-3.31 (m, 1H), 3.89 (s, 3H), 5.74 (d, J=12.0 Hz, 2H), 5.97 (q, J=7.0 Hz, 1H), 7.17 (dd, J=9.0, 9.0 Hz, 2H), 7.20 (dd, J=1.0, 9.0 Hz, 1H), 7.34 (dd, J=6.0, 9.0 Hz, 2H), 7.37 (d, J=1.0 Hz, 1H), 7.60 (d, J=8.0 Hz, 1H), 7.72 (s, 1H), 7.79 (s, 1H), 9.64 (d, J=2.0 Hz, 1H)

### Example 5

### Crystal (II) of 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate

After evaporating the solvent of a methanol (1.0 mL) solution of 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate (53 mg, 0.10 mmol) under a stream of nitrogen, acetonitrile (2 mL) was added to the residue and the mixture was heated to 60°C. The solution was cooled to room temperature and stirred, and the precipitated crystals were collected by filtration. The crystals were washed with heptane and dried under reduced pressure to give the title compound in the form of white crystal.
¹H-NMR (600 MHz, DMSO-d₆) δ (ppm): 1.50 (d, J=7.0 Hz, 3H), 1.58-1.62 (m, 1H), 1.74-1.78 (m, 1H), 2.41 (s, 3H), 2.75-2.79 (m, 2H), 2.86-2.90 (m, 1H), 3.27-3.31 (m, 1H), 3.89 (s, 3H), 5.74 (d, J=12.0 Hz, 2H), 5.97 (q, J=7.0 Hz, 1H), 7.17 (dd, J=9.0, 9.0 Hz, 2H), 7.20 (dd, J=1.0, 9.0 Hz, 1H), 7.35 (dd, J=6.0, 9.0 Hz, 2H), 7.37 (d, J=1.0 Hz, 1H), 7.59 (d, J=8.0 Hz, 1H), 7.73 (s, 1H), 7.79 (s, 1H), 9.63 (d, J=2.0 Hz, 1H)

### Example 6

### Crystal III of 1-{4-[(E)-{1-[(1S)-1-(4-fluorophenyl)ethyl]-2-oxopiperidin-3-ylidene}methyl}-2-methoxyphenyl}-4-methyl-1H-imidazol-3-iomethyl monohydrogen phosphate

Acetone (11 mL) was added with stirring to a 1-propanol (2.0 mL) solution of lyophilized 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate (305 mg, 0.576 mmol). After observing the precipitation of the crystal, acetone (3.0 mL) was further added to the solution. After stirring the solution for 10 minutes, the crystals were collected by filtration. The crystals were washed with a mixed solvent of 1-propanol:acetone = 1:7 and dried under reduced pressure to give the title compound in the form of white crystal (277 mg, 0.523 mmol, 90.8% yield). The obtained compound had the following properties.
¹H-NMR (400 MHz, CD₃OD) δ (pom): 1.59 (d, J=7.2 Hz, 3H), 1.63-1.90 (m, 2H), 2.53 (s, 3H), 2.78-2.85 (m, 2H), 2.95-3.04 (m, 1H), 3.35-3.42 (m, 1H), 3.96 (s, 3H), 5.88 (d, J=12. 8 Hz, 2H), 6.09 (q, J=7.2 Hz, 1H), 7.10 (t, J=8.8 Hz, 2H), 7.21 (dd, J=8.0, 1.2 Hz, 1H), 7.31 (d, J=1.2 Hz, 1H), 7.38 (dd, J=8.8, 5.2 Hz, 2H), 7.59 (d, J=8.0 Hz, 1H), 7.65 (d, J=1.6 Hz, 1H), 7.80 (s, 1H), 9.38 (d, J=1.6 Hz, 1H).

### Example 7

### Crystal (IV) of 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate

Acetone (6.5 mL) was added with stirring to an aqueous solution (0.5 mL) of the 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate (255 mg, 0.482 mmol) obtained in Example 6. After observing the precipitation of the crystal, acetone (3.0 mL) was further added to the solution. After stirring the solution for 10 minutes, the crystals were collected by filtration. The crystals were washed with a mixed solvent of water: acetone = 1:19 and dried under reduced pressure to give the title compound in the form of white crystal (225 mg, 0.425 mmol, 88.2% yield). ¹H-NMR of the compound corresponded with that of Example 6.

### Example 8

### Crystal (V) of 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate

After evaporating the solvent of a methanol (1.0 mL) solution of 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate (49.4 mg, 0.09 mmol) under a stream of nitrogen, acetonitrile (2 mL) was added to the residue. The solution was stirred at room temperature and the precipitated crystals were collected by filtration. The crystals were washed with acetonitrile and dried under reduced pressure to give the title compound in the form of white crystal.
¹H-NMR (600 MHz, DMSO-d₆) δ (ppm): 1.50 (d, J=7.0 Hz, 3H), 1.58-1.62 (m, 1H), 1.74-1.78 (m, 1H), 2.41 (s, 3H), 2.75-2.79 (m, 2H), 2.86-2.90 (m, 1H), 3.27-3.31 (m, 1H), 3.89 (s, 3H), 5.73 (d, J=12.0 Hz, 2H), 5.97 (q, J=7.0 Hz, 1H), 7.17 (dd, J=9.0, 9.0 Hz, 2H), 7.20 (dd, J=1.0, 9.0 Hz, 1H), 7.35 (dd, J=6.0, 9.0 Hz, 2H), 7.37 (d, J=1.0 Hz, 1H), 7.59 (d, J=8.0 Hz, 1H), 7.73 (s, 1H), 7.79 (s, 1H), 9.62 (d, J=2.0 Hz, 1H)

### Measurement of X-ray powder diffraction pattern

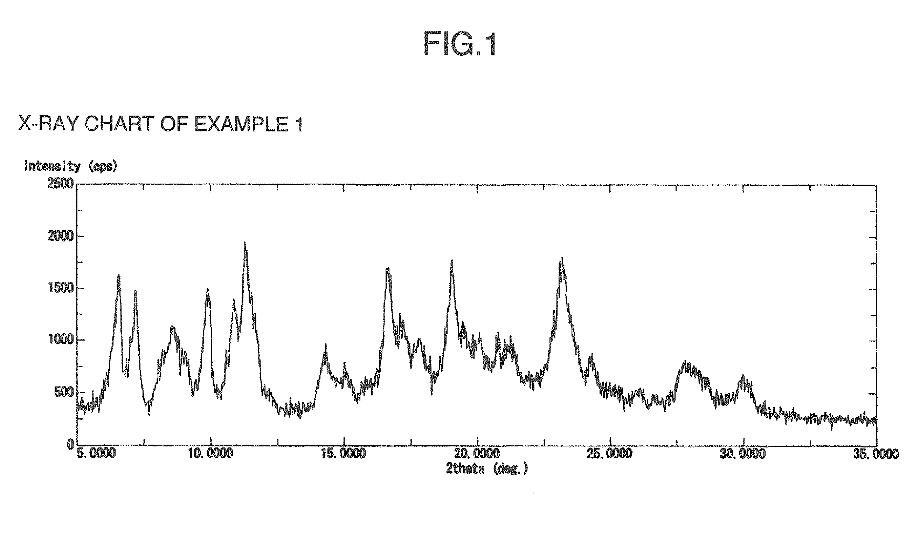

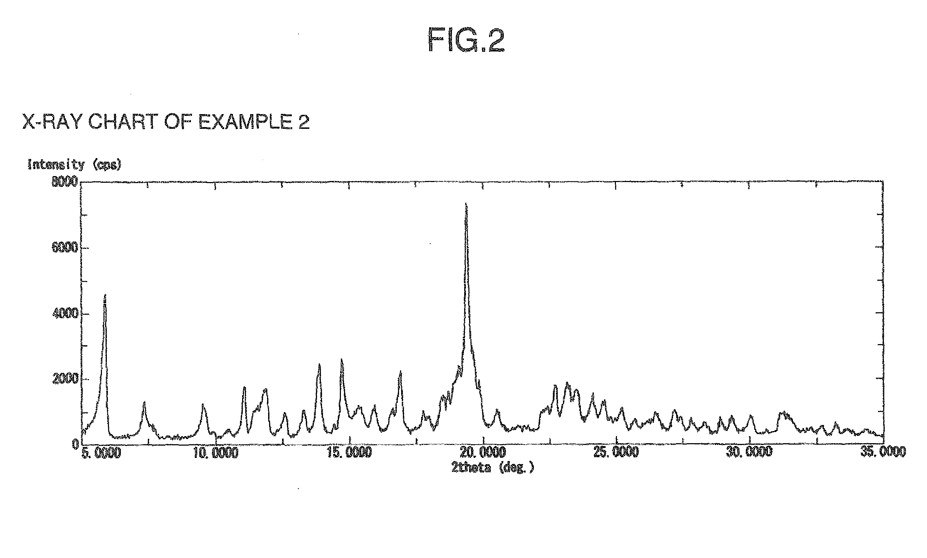

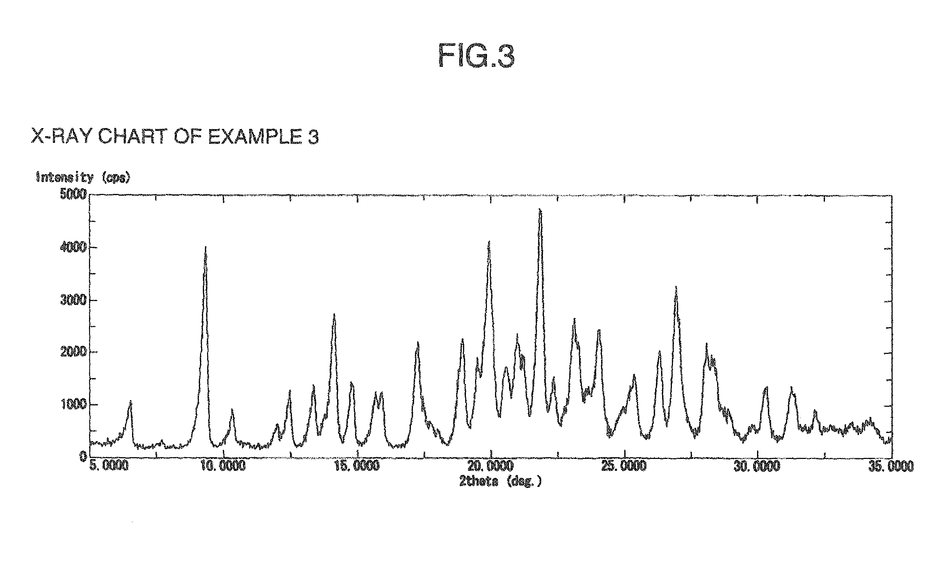

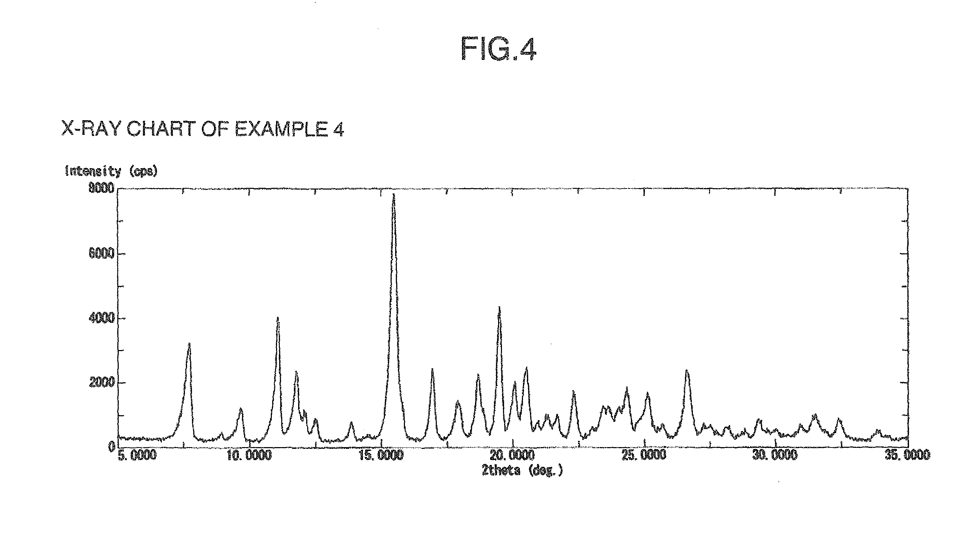

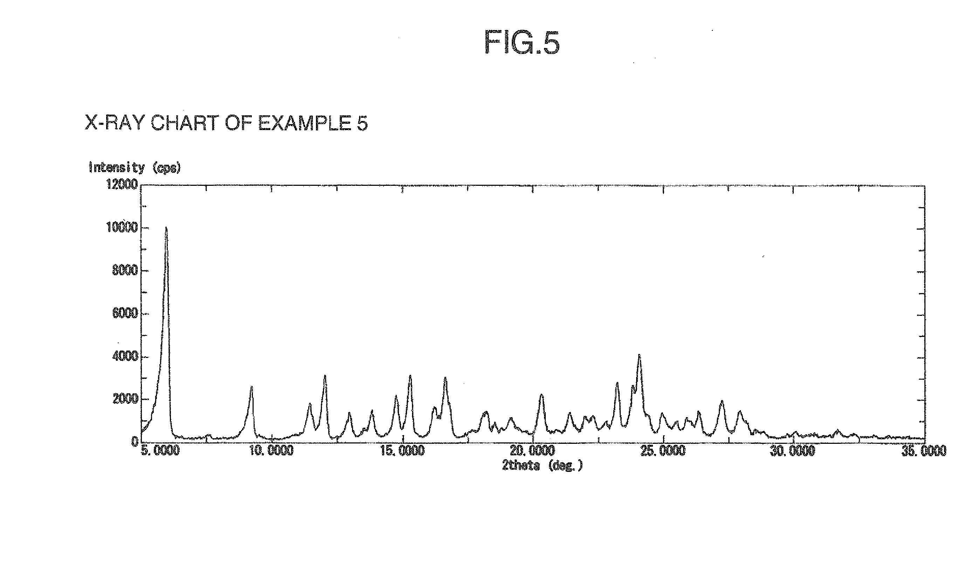

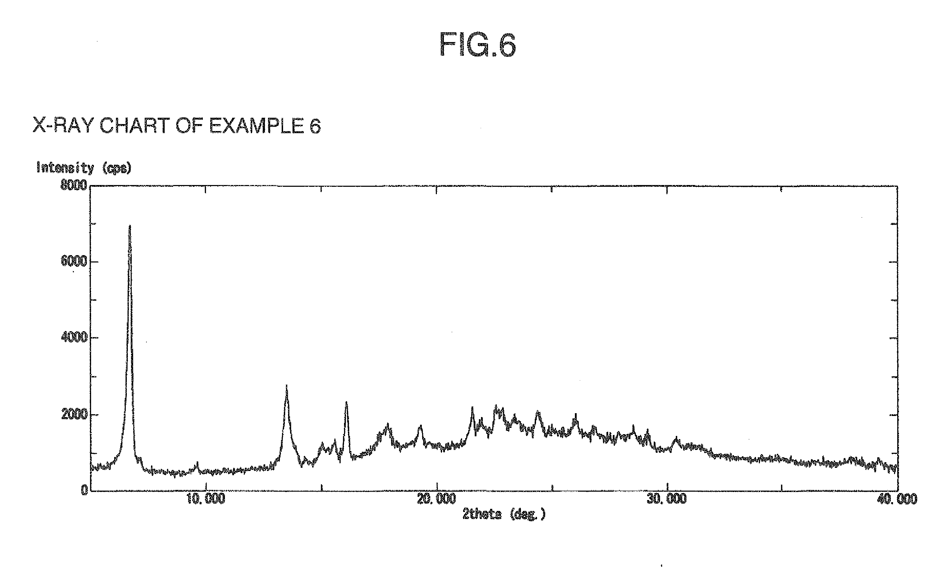

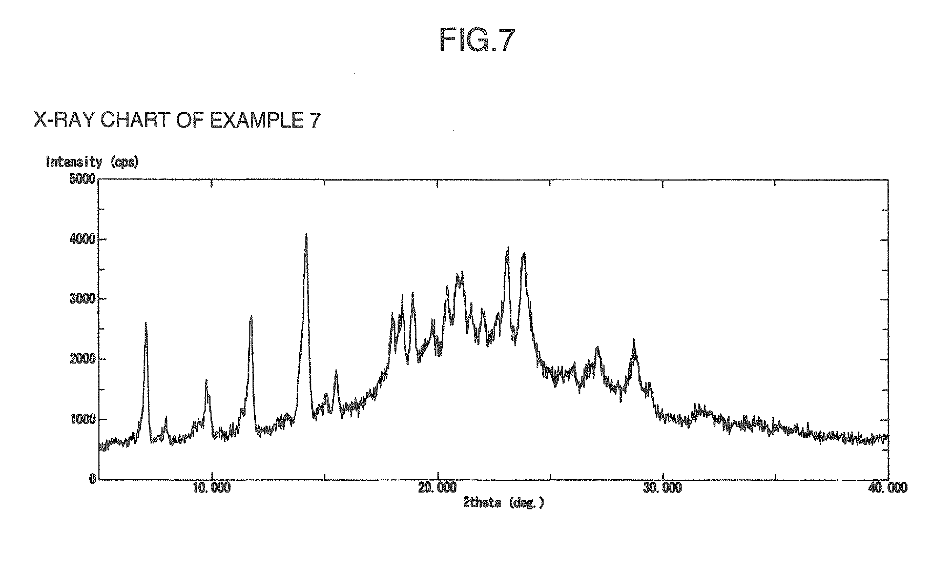

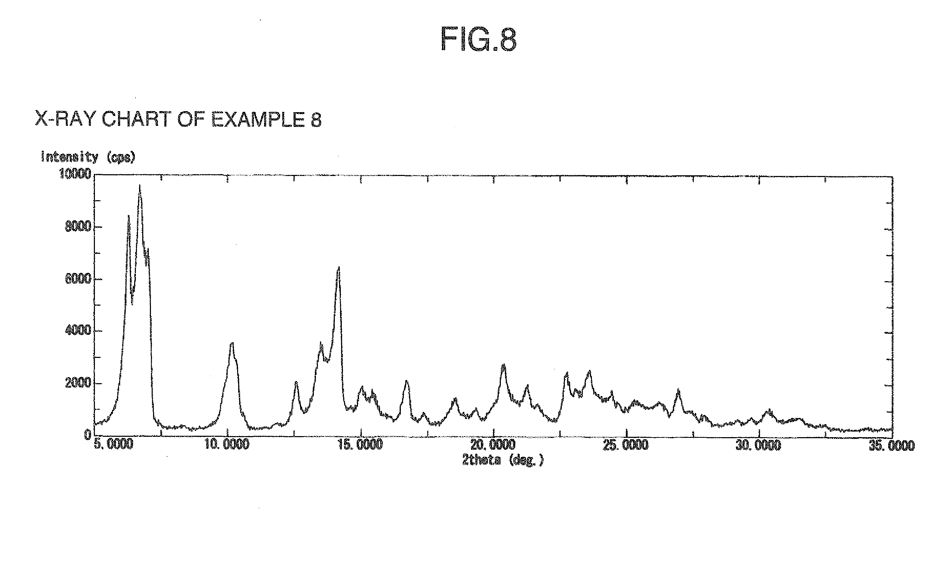

The X-ray powder diffraction patterns for the crystals obtained in each of Examples 1 to 8 were measured under the following conditions. Figs. 1 to 8 show the X-ray powder diffraction pattern of the crystals obtained in each of Examples 1 to 8. Further, Tables 1 to 8 show the distinctive diffraction angle (2θ) peaks of each type of crystal of Examples 1 to 8.

### Measurement conditions in Example 1 to 5 and 8:

Sample holder: aluminium
Target: copper
Detector: scintillation counter
Tube voltage: 50 kV
Tube current: 300 mA
Slit: DS 0.5 mm (Height limiting slit 2 mm), SS Open, RS Open
Scanning speed: 5°/min
Sampling pitch: 0.02°
Scanning range: 5 to 35°
Goniometer: Level goniometer

### Measurement conditions in Examples 6 and 7:

Sample holder: glass
Target: copper
Detector: scintillation counter
Tube voltage: 40 kV
Tube current: 200 mA
Slit: DS 1/2°, SS 1/2°, RS 0.3 mm
Scanning speed: 5°/min
Sampling pitch: 0.02°
Scanning range: 5 to 40°
Goniometer: Perpendicular goniometer

[Table 1]

**Table 1: Representative peaks in the X-ray diffraction pattern of the Example 1 crystal**

| | | 2θ (degree) |
|---|---|---|
| 6.6 | ± | 0.2 |
| 11.3 | ± | 0.2 |
| 16.6 | ± | 0.2 |
| 19.0 | ± | 0.2 |
| 23.2 | ± | 0.2 |

[Table 2]

**Table 2: Representative peaks in the X-ray diffraction pattern of the Example 2 crystal**

| | | |
|---|---|---|
| | | 2θ (degree) |
| 5.9 | ± | 0.2 |
| 13.9 | ± | 0.2 |
| 14.7 | ± | 0.2 |
| 16.9 | ± | 0.2 |
| 19.4 | ± | 0.2 |

[Table 3]

**Table 3: Representative peaks in the X-ray diffraction pattern of the Example 3 crystal**

| | | 2θ (degree) |
|---|---|---|
| 9.3 | ± | 0.2 |
| 14.1 | ± | 0.2 |
| 19.9 | ± | 0.2 |
| 21.8 | ± | 0.2 |
| 23.1 | ± | 0.2 |
| 27.0 | ± | 0.2 |

[Table 4]

**Table 4: Representative peaks in the X-ray diffraction pattern of the Example 4 crystal**

| | | 2θ (degree) |
|---|---|---|
| 7.7 | ± | 0.2 |
| 11.1 | ± | 0.2 |
| 15.5 | ± | 0.2 |
| 19.5 | ± | 0.2 |
| 26.6 | ± | 0.2 |

[Table 5]

**Table 5: Representative peaks in the X-ray diffraction pattern of the Example 5 crystal**

| | | 2θ (degree) |
|---|---|---|
| 6.0 | ± | 0.2 |
| 9.2 | ± | 0.2 |
| 12.0 | ± | 0.2 |
| 15.3 | ± | 0.2 |
| 24.1 | ± | 0.2 |

[Table 6]

**Table 6: Representative peaks in the X-ray diffraction pattern of the Example 6 crystal**

| | | 2θ (degree) |
|---|---|---|
| 6.7 | ± | 0.2 |
| 13.5 | ± | 0.2 |
| 16.1 | ± | 0.2 |

[Table 7]

**Table 7: Representative peaks in the X-ray diffraction pattern of the Example 7 crystal**

| | | 2θ (degree) |
|---|---|---|
| 7.1 | ± | 0.2 |
| 11.7 | ± | 0.2 |
| 14.2 | ± | 0.2 |
| 23.1 | ± | 0.2 |
| 23.8 | ± | 0.2 |

[Table 8]

**Table 8: Representative peaks in the X-ray diffraction pattern of the Example 8 crystal**

| | | 2θ (degree) |
|---|---|---|
| 6.3 | ± | 0.2 |
| 6.7 | ± | 0.2 |
| 10.2 | ± | 0.2 |
| 14.2 | ± | 0.2 |
| 20.4 | ± | 0.2 |

### INDUSTRIAL APPLICABILITY

The present invention can provide the crystal of the prodrug substance of the compound having an Aβ production reducing effect and effective for treating neurodegenerative diseases caused by Aβ such as Alzheimer's disease, or Down's syndrome, or the salt thereof or the salt crystal thereof

### BRIEF DESCRIPTION OF THE DRAWINGS

[Fig.1] Fig. 1 is a drawing showing the X-ray powder diffraction pattern of the crystal obtained in Example 1;
[Fig.2] Fig. 2 is a drawing showing the X-ray powder diffraction pattern of the crystal obtained in Example 2;
[Fig.3] Fig. 3 is a drawing showing the X-ray powder diffraction pattern of the crystal obtained in Example 3;
[Fig.4] Fig. 4 is a drawing showing the X-ray powder diffraction pattern of the crystal obtained in Example 4;
[Fig.5] Fig. 5 is a drawing showing the X-ray powder diffraction pattern of the crystal obtained in Example 5;
[Fig.6] Fig. 6 is a drawing showing the X-ray powder diffraction pattern of the crystal obtained in Example 6;
[Fig.7] Fig. 7 is a drawing showing the X-ray powder diffraction pattern of the crystal obtained in Example 7; and
[Fig.8] Fig. 8 is a drawing showing the X-ray powder diffraction pattern of the crystal obtained in Example 8.

## Claims

1. A salt of 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate.

2. The salt according to claim 1 which is an organic acid salt.

3. The salt according to claim 1 or 2 which is fumarate.

4. The salt according to claim 1 or 2 which is maleate.

5. The salt according to claim 1 which is an inorganic acid salt.

6. The salt according to claim 1 or 5 which is hydrobromate.

7. A crystal of fumarate according to claim 3 having a peak at a diffraction angle (26±0.2°) of 11.3°, 19.0° and/or 23.2° in the X-ray powder diffraction peak.

8. A crystal of maleate according to claim 4 having a peak at a diffraction angle (28±0.2°) of 5.9°, 14.7° and/or 19.4° in the X-ray powder diffraction peak.

9. A crystal of hydrobromate according to claim 6 having a peak at a diffraction angle (26±0.2°) of 9.3°, 19.9° and/or 21.8° in the X-ray powder diffraction peak.

10. A crystal (I) of 1-{4-[(E)-{1-[(1S)-1-(4-fluorophenyl)ethyl]-2-oxopiperidin-3-ylidene}methyl]-2-methoxyphenyl]-4-methyl-1H-imidazol-3-iomethyl monohydrogen phosphate having a peak at a diffraction angle (20±0.2°) of 11.1°, 15.5° and/or 19.5° in the X-ray powder diffraction peak.

11. A crystal (II) of 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate having a peak at a diffraction angle (28±0.2°) of 6.0°, 15.3° and/or 24.1° in the X-ray powder diffraction peak.

12. A crystal (III) of 1-{4-[(E)-{(1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate having a peak at a diffraction angle (28±0.2°) of 6.7°, 13.5° and/or 16.1° in the X-ray powder diffraction peak.

13. A crystal (IV) of 1-{4-[(E)-{1-[(1S)-1-(4-fluorophenyl)ethyl]-2-oxopiperidin-3-ylidene}methyl]-2-methoxyphenyl}-4-methyl-1H-imidazo1-3-iomethyl monohydrogen phosphate having a peak at a diffraction angle (20±0.2°) of 7.1°, 11.7° and/or 14.2° in the X-ray powder diffraction peak.

14. A crystal (V) of 1-{4-[(E)-{1-[(1S)-1-(4-fluorophenyl)ethyl]-2-oxopiperidin-3-ylidene}methyl]-2-methoxyphenyl}-4-methyl-1H-imidazol-3-iomethyl monohydrogen phosphate having a peak at a diffraction angle (20±0.2°) of 6.3°, 6.7° and/or 14.2° in the X-ray powder diffraction peak.
